# EUROPEAN PATENT SPECIFICATION

(11) **EP 0 533 842 B1**
(45) Date of publication and mention of the grant of the patent: **27.12.1995**
(21) Application number: 91912979.1
(22) Date of filing: 10.06.1991
(51) Int. Cl.: B65D 83/10, B65D 85/24, A61M 5/32

(54) **DISPOSAL FOR DISENGAGING AND RECEIVING NEEDLES**
BEHÄLTER ZUM ENTFERNEN UND ENTSORGEN VON SPRITZENNADELN
POUBELLE DE DETACHEMENT ET DE RECEPTION D'AIGUILLES

(30) Priority: 12.06.1990 US 536708; 31.08.1990 US 576378
(43) Date of publication of application: 31.03.1993
(73) Proprietor: MEDICAL SAFETY PRODUCTS, INC., Denver, CO 80222 (US)
(72) Inventor: SAGSTETTER, William, E., Denver, CO 80221 (US); COOKE, John, E., Lakewood, CO 80215 (US)
(74) Representative: Dallmeyer, Georg, Dipl.-Ing.
(86) International application number: US9104084
(87) International publication number: WO9119652

(56) References cited:
- FR-A- 2 624 023
- US-A- 4 375 849
- US-A- 4 466 538
- US-A- 4 494 652
- US-A- 4 667 821
- US-A- 4 738 362
- US-A- 4 798 587
- US-A- 4 801 013
- US-A- 4 802 579
- US-A- 4 807 344
- US-A- 4 917 243
- US-A- 4 923 447
- US-A- 4 986 811
- US-A- 4 995 871
- US-A- 5 024 327
- US-A- 5 031 767

## Description

To obtain a blood sample for diagnostic purposes, blood is drawn into an evacuated blood collection tube through a double ended needle. The needle includes a hub, an anterior needle, for tissue penetration into a blood vessel and a posterior needle having an exterior elastomeric valve. The posterior end of the needle is penetrably inserted within a barrel having female threads for threadedly engaging the hub to retain the double ended needle positionally fixed with respect to the barrel. The proximal end of the barrel is to open to receive an evacuated blood collection tube having a stopper for penetrably receiving the posterior needle. Upon venipuncture, blood will flow through the anterior needle and the posterior needle into the collection tube. Upon removal of the collection tube, the elastomeric valve recovers the posterior needle to prevent spontaneous blood flow from the needle. A phlebotomist can then insert a second or more blood collection tubes into the barrel to receive additional blood samples.

Upon completion of the venipuncture procedure, the anterior needle is withdrawn from the patient. While the barrel is often reused, the double ended needle must be safely removed from the barrel without causing needle stick and while avoiding contact with any residual body fluids of the patient to prevent transmission of infectious disease. Typically, a conventional bio-hazard receptacle for needles (US-A-4,917,243, US-A-4,802,579, US-A-4,667,821) is provided with a lid having various shaped slots to engage the needle hub. To dispose of a used needle, the phlebotomist must carefully place the exposed needle hub into the slot, grip and rotate the barrel to unthread the needle and cause the disengaged needle to drop completely through the slot into the underlying container. Since the barrels are often opaque, it is difficult to know when the double ended needle has become completely threadedly disengaged from the barrel. A further danger arises from the upstanding exposed posterior needle until the hub has become sufficiently disengaged from the slot to permit the needle to drop into the receptacle. Aside from hub engaging slots, other devices have been developed including the use of fixed and moveable jaws to engage the needle hub. Mechanized devices for unthreading a double ended needle are also known.

US-A-4,917,243, US-A-4,802,579 and US-A-4,667,821 describe an urging means but the urging means involves the user performing an extra step to remove the needle from the holder device. In US-A-4,917,243 the user must cause the edge of hub to grasp 5 to remove needle from hub. In US-A-4,802,579, the user must move the disconnected needle down into larger opening 14 to allow it to fall into the container. In US-A-4,667,821, the user must move the needle toward the wide portion 22 of the slot to allow it to fall into the container.

A recently available reusable safety blood collection device includes a holder for engaging the double ended needle, which holder is translatable within a guard to fully enclose and shield both the anterior and posterior needles of the double ended needle. The guard includes an anterior collar for shielding the end of the anterior needle upon retraction of the holder and for supporting therewithin the hub engaging boss of the holder during use. Known syringe disposal devices are not well suited for receiving and disposing needles of such devices since the hub of the double ended needle is shielded by the collar and is not accessible for griping by the opposed edges of a slot, jaws or the like.

It is therefore an object of the present invention to provide an apparatus and a method for receiving and safely disengaging a double ended needle from a blood collection tube holder on completion of a venipuncture procedure while shielding a clinician from exposure to each end of the needle.

This object is achieved by the invention according to claims 1 and 11.

A post mounted within a receptacle interferingly engages a rib of a conventional double ended needle hub to prevent rotation of the hub upon engagement of a rib. A sloping upper surface of the post encourages downward sliding movement of the needle into the receptacle upon threaded disengagement of the hub. In a second embodiment, a partial annular slot receives the collar of a guard for an enclosed double ended needle supporting holder and a post extending from the slot interferingly engages with a hub rib. Rotation of the guard with commensurate rotation of the enclosed holder will threadedly disengage the double ended needle from the holder whereafter the needle will drop into the receptacle. To encourage drop of the double ended needle, a pair or vertical diametrically opposed leaf springs may be incorporated, which leaf springs includes a lip for preventing upward withdrawal of the double ended needle. In a variant, the hub engaging and supporting post assembly along with a downwardly directed chute for guiding a double ended needle into a receptacle nay be of modular construction attachable to an aperture of any container.

A post is provided for threadedly disengaging a double ended needle from a blood collection tube holder.

Furtheron a post for engaging a rib of the hub of a double ended needle can be provided to permit unthreading the needle from a blood collection tube holder in combination with a pair of leaf springs to encourage dropping of the needle into an underlying receptacle.

An annular slot can be provided for receiving a collar of a blood collection tube holder supporting guard, which collar is concentric with a post for interferingly engaging a rib of a double ended needle hub threadedly engaged with the holder.

Furtheron a receptacle mounted post can be provided for engaging a rib of a double ended needle hub to permit threaded disengagement of the hub from a supporting blood collection tube holder and to encourage drop of the needle into the post supporting receptacle.

A transparent disposal for receiving a double ended needle from a transparent blood collection tube holder may be provided to permit visual inspection of the separation and disposal of the needle.

The present invention will be described with greater clarity and specificity with reference to the following drawings, in which:
Figure 1 is an isometric view of a container supporting posts for engaging the hubs of double ended needles threadedly engaged with different types of blood collection tube holders;
Figure 2 is a partial top view of two types of post assemblies mounted in a container for receiving used doubled needles;
Figure 3 is a partial sectional view illustrating the relationships between needle hub engaging devices and the respective types of blood collection tube holders;
Figure 4 illustrates a variant of the hub engaging post structure;
Figure 5 is a partial cross sectional view illustrating operation of the variant shown in Figure 4;
Figure 6 is an isometric view of a variant of the needle hub engaging post and associated structure;
Figure 7 is a cross sectional view taken along lines 7-7, as shown in Figure 6;
Figure 8 is a cross sectional view taken along lines 8-8, as shown in Figure 7;
Figure 9 is a top view taken along lines 9-9, as shown in Figure 8;
Figure 10 is a top isometric view of a further variant;
Figure 11 is a top view of the further variant;
Figure 12 is a cross sectional view taken along lines 12-12, as shown in Figure 11; and
Figure 13 is an exploded-view illustrating a yet further variant of the present invention and mounting means therefore.

### Description of the Preferred Embodiment

Receptacles for used blood collection tube holders with double ended needles of the type associated with venipuncture procedures have been in existence for a period of years. Such receptacles tend to reduce the likelihood of needle stick and spread of infectious disease resulting from contact with the needles and body fluids disposed upon and within the needles. When blood collection tube holders used with the needles are not to be disposed, various problems arise in attempting to separate the double ended needle from the holder without requiring a phlebotomist to touch the needle. A number of devices for this purpose have been developed but each suffers from actual or potential problems which preclude repetitive fail safe operation.

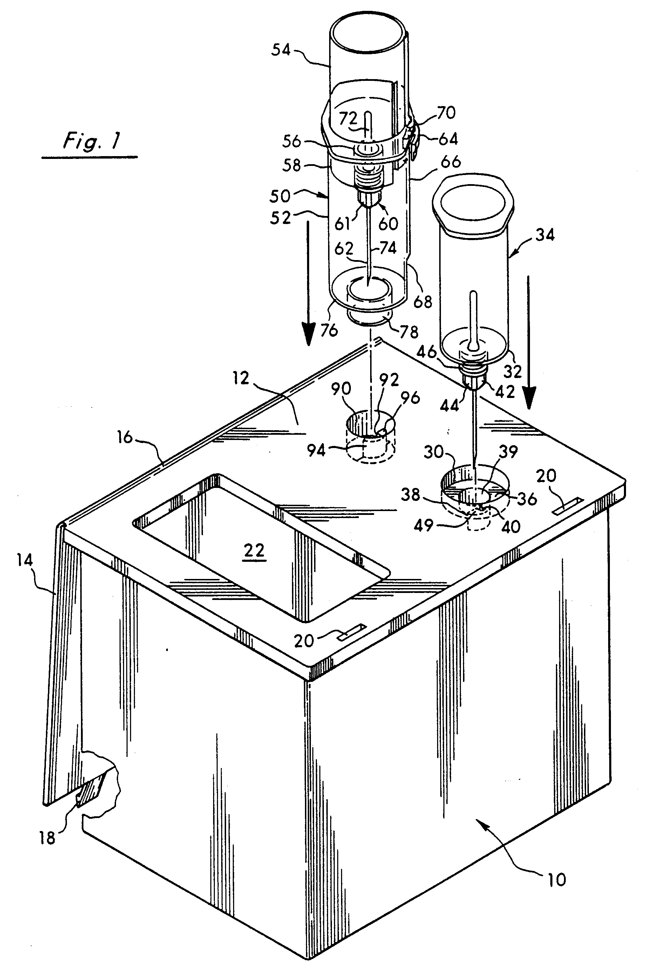

Referring to Figure 1, there is illustrated a receptacle 10 for receiving and housing used double ended needles. The receptacle includes a top surface 12 and a cover 14. The cover may be hinged along hinge line 16. Prongs, such as prong 18 may extend from cover 14 for locking engagement with slots, such as slots 20 formed in top surface 12. An opening 22 may be formed in the top surface to permit insertion into the receptacle of various items for disposal.

Referring jointly to Figures 1, 2 and 3, a circular recess 30 is formed in top surface 12 to receive and nestingly support cylindrical anterior end 32 of a conventional blood collection tube holder 34. An apertured supporting surface 36, which may be partially or completely annular, is disposed at the bottom end of recess 30 to support anterior end 32 of the blood collection tube holder. A post 38, oriented off center within recess 30, includes a slot 40 for receiving a rib 42 formed in hub 44 of a conventional double ended needle threadedly engaged with boss 46 formed in anterior end 32 of the blood collection tube holder.

To eliminate the possibility of needle stick by phlebotomists during a conventional venipuncture procedure, a more complex blood collection tube holder device 50 has been developed. This device includes a barrel 52 for receiving and supporting a holder 54. The holder includes a boss 56 disposed at anterior end 58 for threadedly engaging hub 60 of a conventional double ended needle 62. A spring loaded tab 64 extends from anterior end 58 of holder 54 for penetrable engagement with and translation along a slot 66 formed longitudinally in barrel 52. Preferably, the slot includes laterally expanded anterior segment 68 and posterior segment 70 for lockingly receiving the tab to retain holder 54 locked in the anterior or posterior position. The axial length of holder 54 is sufficient to fully enclose posterior needle 72 to prevent inadvertent contact with the needle. Upon translation of holder 54 to its posterior position, anterior needle 74 is enclosed within the anterior portion of barrel 52. Anterior end 76 of barrel 52 includes a collar 78 for accommodating penetration therethrough of anterior needle 74 and at least a portion of hub 60 of double ended needle 62. Upon retraction of holder 54 to its posterior position, the end of anterior needle 74 may be located within the confines of collar 78.

To accommodate disengagement of double ended needle 62 from holder 54 on completion of a venipuncture procedure, receptacle 10 may be used. A cylindrical passageway 90 is sized and configured to receive and guide collar 78 through top surface 12 of the receptacle. The passageway also serves the function of stablizing device 50 during the process of unthreading double ended needle 62 from holder 54. After placement of collar 78 within passageway 90, tab 64 is translated along slot 66 to position hub 60 within collar 78. Simultaneously, a rib 61 of the hub will slidingly engage slot 92 disposed in post 94 located within passageway 90. The size and orientation of post 94 permits the post to extend into collar 78 of device 50 to accomodate engagement with the hub 60.

To disengage the double ended needle from blood collection tube holder 34, the holder is inserted into recess 30, guiding the anterior needle 48 through aperture 39 downwardly into adjacent slot 40 until rib 42 of hub 44 slidingly engages the slot. In this position, anterior end 32 of the holder will rest upon and be supported by supporting surface 36. Upon counterclockwise rotation of the holder, commensurate rotation of hub 44 will be procluded by interference between rib 42 and slot 40, resulting in unthreading of the hub from the holder. Upon subsequent raising of the holder out of the recess, the hub, will slide downwardly into the receptacle through the space between post 38 and supporting surface 36. Such downward sliding movement is encouraged if top surface 49 of the post is canted downwardly toward slot 40.

To separate double ended needle 62 from holder 54 of device 50, collar 78 is inserted within passageway 90 until anterior end 76 of barrel 52 rests upon top surface 12 of receptacle 10. Thereafter, tab 64 is brought out of detent or expanded segment 70 and translated along passageway 66 until rib 61 of hub 60 engages slot 92 in post 94. Subsequent counterclockwise rotation of barrel 52 will result in commensurate rotation of holder 54 due to interference therebetween provided by tab 64 and slot 66 or further detent or expanded segment 68. The counterclockwise movement will unthread hub 60 from boss 56. Prior to or upon lifting of device 50, disengaged double ended needle 62 will drop through the space intermediate post 94 and passageway 90. Downward sliding movement of the double ended needle will be enhanced if top surface 96 of post 94 cants downwardly toward the slot.

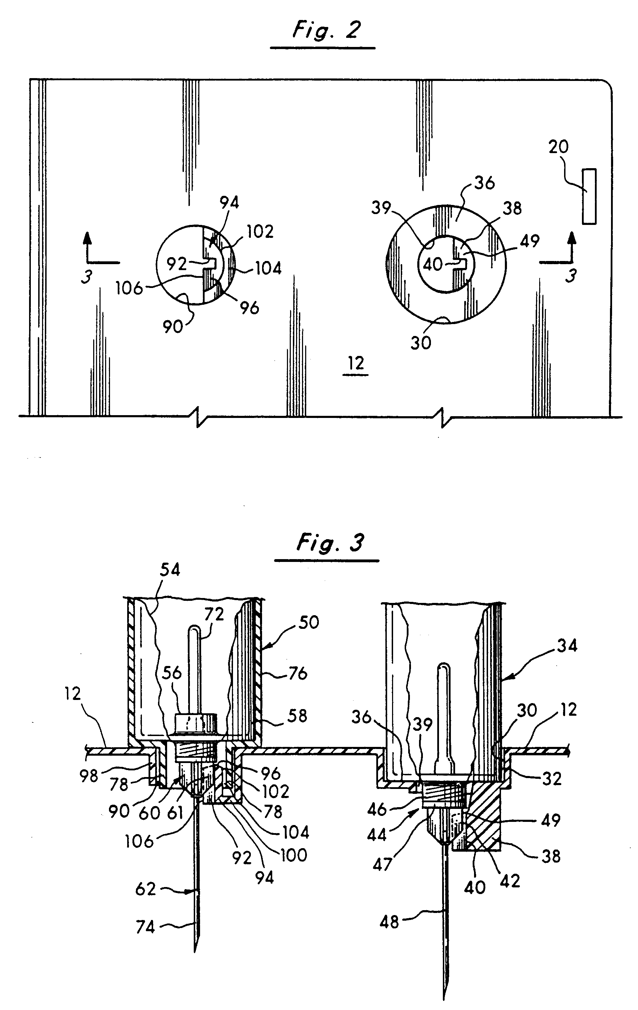

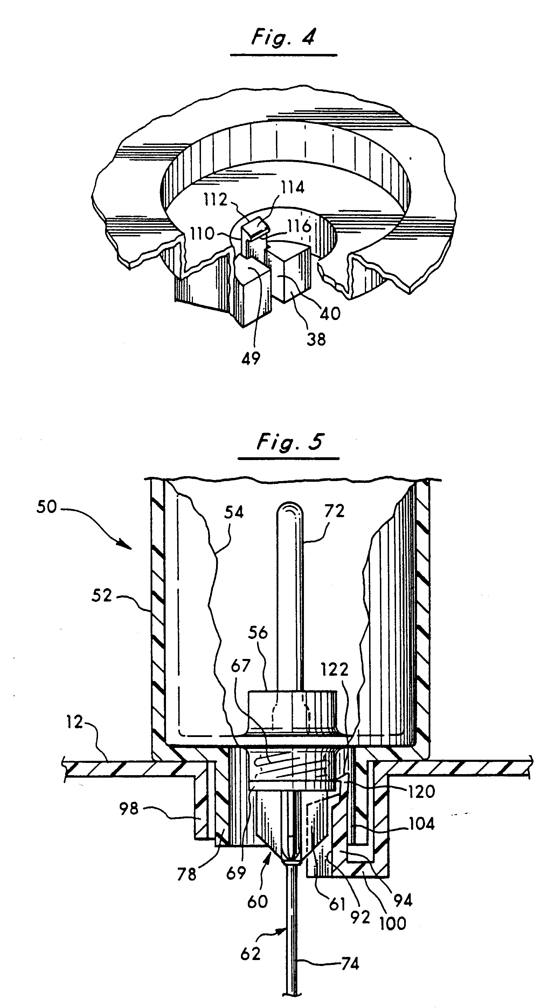

Referring jointly to Figures 2 and 3, further details attendant the needle receiving elements of receptacle 10 will be described. Post 38 attendant recess 30 depends from a segment of supporting surface 36. By incorporating recess 30, anterior end 32 of holder 34 is reasonably well guided and supported during rotation of the holder to prevent skewing of the double ended needle upon partial unthreading and the act of unthreading will be enhanced. Moreover, the recess will maintain the holder in axial alignment with slot 40 to ensure continuing engagement of rib 42 of the hub with the slot. Top surface 49 of post 38 is necessarily dropped below supporting surface 36 to an extent sufficient to accommodate the extending axial dimension of boss 46 and the axial positioning of a band 47 normally found in hub 44, which band segregates the ribbed portion of the hub from the threaded portion of the hub. As noted above, a downward canting of top surface 49 is preferable to encourage sideways movement of a freed double ended needle to assist the hub in clearing the post prior to dropping into the receptacle 10. As particularly noted in Figure 2, post 38 may extend across a chord of aperture 39 defined by supporting surface 36. Such configuration will assist in locating the hub of the needle with respect to slot 40 and minimize the likelihood of the hub missing or not engaging the post in the manner intended.

Passageway 90 includes and is defined by a depending annular skirt 98, which skirt guides and stabilizes collar 78 upon mounting of device 50. A shelf 100 extends centrally into passageway 90 from skirt 98, which shelf supports post 94. The post may include a curved side 102 concentric with passageway 90 to define therebetween an annular slot 104 for receiving a segment of collar 78 of device 50. Slot 92 may extend inwardly from a flat surface 106 interconnecting opposed edges of curved side 102.

As will be evident by inspection, annular slot 104 in combination with the remaining surface area of skirt 98 defining passageway 90 will permit unimpeded rotation of device 50 about its longitudinal axis (and double ended needle 62) but generally impede pivoting or lateral displacement of the device. The limited permissible movement of the device will encourage non binding interference between the rib engaged with slot 92 and permit ready disengagement upon dropping of the double ended needle after threaded disconnection between hub 60 and boss 56.

Referring to Figure 4 there is shown a variant structure for either or each of posts 38, 94. Under abnormal conditions, a double ended needle may not disengage from the supporting post and drop into receptacle 10 as intended and expected. To encourage lateral movement of the hub of the respective double ended needle off of the top surface of the respective post and to totally eliminate any basis for support for the double ended needle, bias means are incorporated to force the needle out of engagement with the post. A bias means of this type is illustrated in Figure 4. A leaf spring 110 is disposed at the rear of a slot, such as slot 40, of one of the posts, such as post 38. This leaf spring may be formed as part of the post with its lower end extending upwardly from the lower part of the post. As illustrated, the leaf spring may replace the wall portion of the post directly rearwardly of the respective slot; the leaf spring may be formed in place of a part of the wall portion depicted in Figures 2 and 3; or, the leaf spring may be separate from the post. By having leaf spring 110 extend forwardly in its relaxed state, it will be forced rearwardly upon engagement of a double ended needle hub with the post. Accordingly, the leaf spring will bear against and bias the hub out of the slot. Upon disengagement of the double ended needle from the blood collection tube holder, the double ended needle will no longer be positionally restrained and the bias urged by the leaf spring will be exercised. Such exercise will cause the double ended needle to be urged out of engagement with the slot. Leaf spring 110 thereby contributes to release of the double ended needle to permit it to drop under force of gravity into the underlying receptacle; the drop may be augmented by the leaf spring. As depicted in Figure 4, top surface 49 may be canted to further encourage disengagemnt between the double ended needle and the post.

Conventional double ended needless include a band disposed about the hub intermediate the threaded segment and the ribbed segment, as illustrated in Figure 3. Under certain circumstances, despite threaded disengagement between a blood collection tube holder and a double ended needle, the double ended needle may remain attached to the boss of the blood collection tube holder, the double ended needle may inadvertently be withdrawn from receptacle 10 and later drop somewhere else. To prevent this from happening, leaf spring 110, as shown in Figure 4, includes a lip 112 for bearing against the upper edge of the band attendant the hub of the double ended needle. Because of the bias provided by the leaf spring, the lip will be urged toward the hub for such engagement. To permit passages of the band past the lip upon insertion of the double ended needle, a ramp 114 may be formed to force the lip laterally as the band passes therepast. The resulting sharp edge 116 will assist repositioning of the lip adjacent the top edge of the band of the hub upon initially unthreading of the hub. Upon upward movement of the holder due to threaded release of the double ended needle, the lip, bearing against the upper edge of the band about the hub, will then move over the top surface of the band and restrain upward movement of the double ended needle. Such restraint will be enough to completely disengage the needle from the blood collection tube holder. Once complete disengagement is effected and with the aid of the leaf spring mounted lip, the double ended needle is free to drop into the receptacle, as discussed above.

A further structural configuration of leaf spring 110 with its lip 122 is depicted in Figure 5. Post 94, as also shown in Figure 3, includes a slot 92 for receiving rib 61 of hub 60 in threaded engagement with boss 56 of device 50. The hub includes an annular band 69 disposed intermediate the plurality of ribs and threaded segment 67. Bias means, which may be a leaf spring 120, extends upwardly from post 94 laterally of slot 92. The upper end of the leaf spring includes a lip 122 extending from the leaf spring toward the slot. The vertical position of lip 122 is set to permit band 69 to be placed intermediate the top of post 94 and the lip. Furthermore, the orientation of the leaf spring, in combination with the extent of lip 122, is established to ensure overlap of the lip with the band upon engagement of hub 60 with post 94. Upon such initial engagement, the band may cause the leaf spring to spring rearwardly (laterally) to permit the band to clear the lip. Upon further downward movement of the band, the leaf spring will cause the lip to translate forwardly (laterally) into an overlying engagement with the band. Subsequent upward movement of hub 60 would result in interference between band 69 and lip 122 to discourage further upward movement. Thereby, lip 122 in combination with its supporting leaf spring 120, will encourage complete disengagement between double ended needle 62 and boss 56. Alternatively, a single bendable lip or lips may extend radially inwardly to engage the hub and prevent withdrawal of the needle.

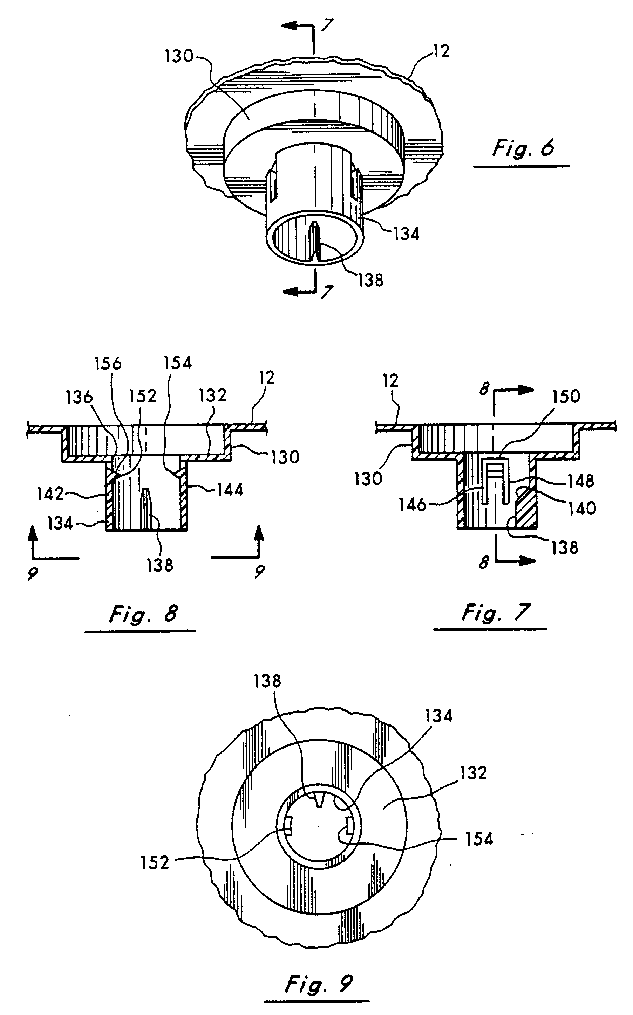

Referring jointly to Figures 6, 7, 8 and 9, a cylindrical wall 130 depends from top surface 12 to define a cylindrical recess for receiving end 32 of a blood collection tube holder, such as holder 34 shown in Figure 1. An annular base 132 extends radially inwardly from the cylindrical wall to support the holder. A cylindrical skirt 134 depends from the annular base proximate aperture 136 formed centrally of the annular base. The interior diameter defined by cylindrical wall 130 is approximately equivalents to that of end 32 of the blood collection tube holder. The interior diameter of cylindrical skirt 134 is approximately that defined by hub 44 (see Figure 1) double ended needle 62. As discussed above, hub 44 includes a plurality of ribs, usually equiangularly spaced, extending longitudinally along the hub. A longitudinally oriented post 138 extends radially inwardly from cylindrical skirt 134. The extent of radial inward extension of post 138 is sufficient to interferingly engage with one of the ribs on hub 44 upon insertion of end 32 of the blood collection tube holder within the recess defined by cylindrical wall 130. Thus, post 138 will interferingly engage a rib of the hub to prevent rotation of the hub commensurate with rotation of the holder;
thereby, threaded disengagement of the hub from the holder may be effected.

To encourage drop of double ended needle 62 (Figure 1) into receptacle 10 upon threaded disengagement with the holder, the end of the post may be canted inwardly downwardly to serve in the manner of a ramp 140 to encourage downward sliding movement of the double ended needle. Sometimes, due to manufacturing tolerances or for other reasons, disengagement between the double ended needle and the holder may require application of a force more positive than that of gravity. To ensure disengagement after unthreading, a pair of diametrically opposed leaf springs 142, 144 may be formed in cylindrical skirt 134. These leaf springs may be defined by a pair of slots 146, 148 joined at the upper end by a third slot 150. Thus, the lower end of each leaf spring is formed as a part of the cylindrical skirt while the upper end is free to flex. The interior upper end of leaf spring 142 includes an inwardly directed lip 152. The radial inward extension of lip 152 is sufficient to engage the top edge of the needle hub and interfere with upward withdrawal of the hub upon upward movement of the holder. Leaf spring 144 includes a similar lip 154. Upper surfaces 156, 158 may be canted inwardly downwardly to accomodate passage therepast of the hub upon insertion of the blood collection tube holder within cylindrical wall 130. During such insertion, the leaf springs will cant radially outwardly to accommodate transport of the hub past lips 152, 154.

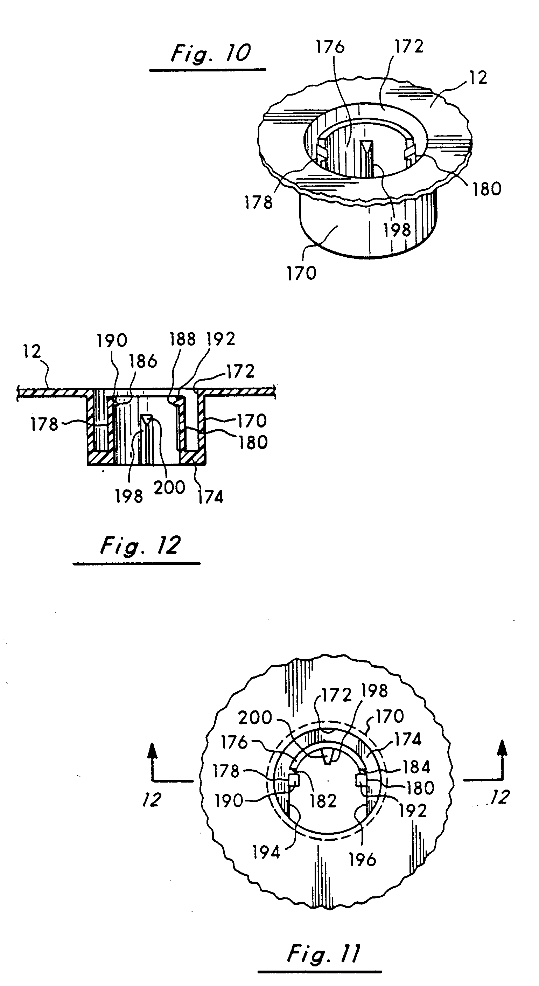

Referring jointly to Figures 10, 11 and 12, there is shown a further variant for receiving a more complex blood collection tube holder device 50, as shown in Figure 1. A cylindrical wall 170 extends downwardly from top surface 12 and defines an aperture 172. A base 174 extends radially inwardly from a part of the lower end of the cylindrical wall. In particular, the base defines a semi annular segment for supporting an upwardly extending semi cylindrical flange 176. The flange is radially inwardly displaced from the interior surface of cylindrical wall 172 to form a slot having a radial width commensurate with the radial width of collar 78 of blood collection tube device 50 (see Figures 1 and 3). The longitudinal extremities ,of the semi cylindrical flange include leaf springs 178, 180. These leaf springs may be formed as part of the semi cylindrical flange and defined by slots 182, 184. The upper ends of the leaf springs may include radially inwardly oriented lips 186, 188. The top of these lips may be canted downwardly inwardly to define ramps 190, 192. Base 174 may be terminated by edges 194, 196, which edges extend tangentially from leaf springs 178, 180 to cylindrical wall 170. A post 198 extends radially inwardly from semi cylindrical flange 176 at its approximate mid point. The upper end of the post may be canted inwardly downwardly to define a ramp 200.

In operation, collar 78 (see Figure 3) of blood collection holder device 50 is inserted within the slot defined by cylindrical wall 170 and semi cylindrical flange 176. Support for the blood collection tube holder device may be provided by the holder resting on surface 12 and by collar 78 resting upon base 174 or either one. The inward extension of post 198 is sufficient to extend adjacent the body of hub 60 and intermediate ribs 61 of the hub to interferingly engage a rib in the event of rotation of the blood collection tube device about its longitudinal axis. Thereby, rotation of the blood collection tube device will result in unthreading and disengagement of the hub supported double ended needle from the blood collection tube device. Upon withdrawal of the blood collection tube device, the hub and its needle will drop into receptacle 10. To ensure disengagement of the hub, lips 186, 188 of leaf springs 178, 180 are vertically positioned and radially inwardly extended to contact the upper end of the hub. With such contact, a resistive force will be exerted upon the hub to prevent upward translation of the hub in response to upward movement of the blood collection tube device. Thereby, disengagement of the hub supporting needle will be assured. By forming ramp 200 upon the post, the upper end of the post will have little support for the hub and the hub will slide off the post. By forming the upper surfaces of leaf springs 186, 188 with ramps 190, 192, lateral outward displacement of the leaf springs to accommodate downward passage therepast of the hub is assured.

By inspection, it will be evident that the retention and positioning of the collar of the blood collection tube holder device intermediate semi cylindrical flange 176 and the interior wall of cylindrical wall 172, little lateral movement of the blood collection tube holder device will result; thus, interfering engagement by post 198 with the ribs of the hub is assured.

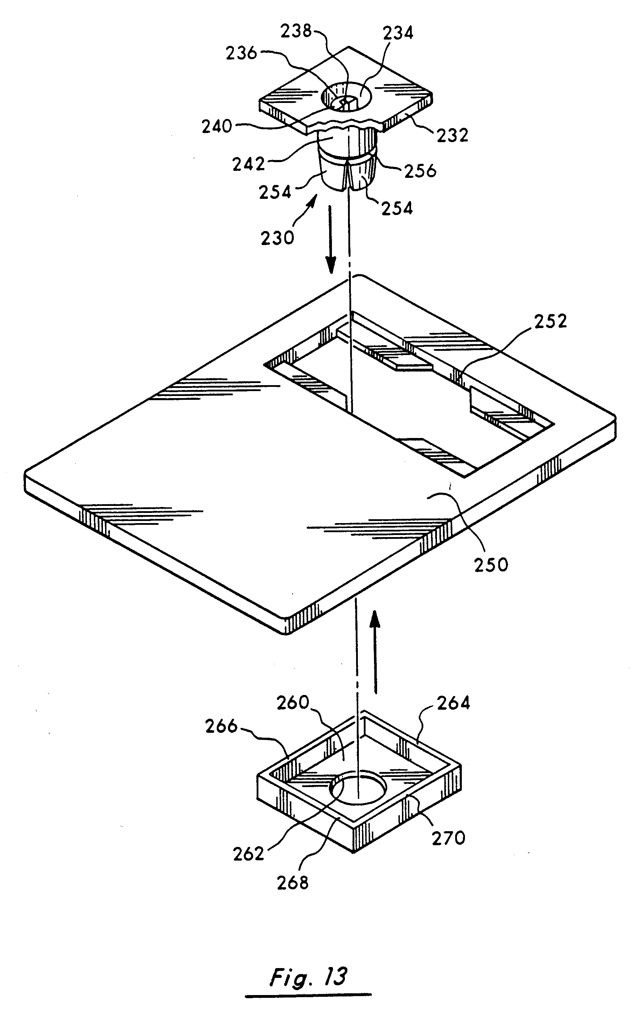

Figure 13 illustrates a variant of the present invention usable in conjunction with any container or receptacle having a lid, which receptacle is to be employed for receiving used double ended needles. A module 230 includes a plate 232 having a recess 234 formed therein; this recess may be of the type shown in Figure 3 for use with a blood collection device 50 or of the type shown in either of Figure 6 or 10. As described above, a post 236 having a slot 238 is disposed within recess 234. Top surface 240 of post 236 may be horizontal, as depicted, or may be canted, as shown in Figure 3; alternatively, the post may be of the type described with respect to Figure 6 to 10. A circular shroud 242 extends downwardly from plate 232. This shroud serves in the manner of a guide or chute to direct disengaged double ended needles therethrough.

Module 230 is to be used in conjunction with a receptacle having an apertured top for penetrably receiving shroud 242. Thereby, any receptacle can be used as a disposal unit for double ended needles upon attachment of module 230. The means for retaining the module in place may be permanent or temporary.

A top 250 of a presently widely used receptacle is depicted. The top includes an aperture 252 of sufficient size to permit penetrable engagement by shroud 242. The length and width of plate 232 supports module 230 upon top 250 after penetration of shroud 242. The lower end of the shroud may include a plurality of outwardly biased resilient wings 254 extending downwardly from a groove 256 formed in the shroud. Locking means, such as plate 260, includes an aperture 262 dimensioned to be seated in groove 256.

In operation, module 230 is penetrably engaged with aperture 252 of top 250. Protruding wings 254 are forced radially inwardly to pass through aperture 262 in plate 260 and the plate is translated upwardly along shroud 242 until aperture 262 seats within groove 256. Thereafter, wings 254 will tend to spread radially outwardly to prevent disengagement of plate 260. The module will now be locked in place. Plate 260 may include upwardly extending sidewalls 264, 266, 268 and 270 to provide dimensional correspondence between the extent to which groove 256 is below top 250 with the position of aperture 262 of plate 260 to preclude vertical movement of the module and to frictionally maintain the module at a predetermined location within aperture 252.

Even though top 250 having a particularly configured aperture 252 corresponds with a widely used type of receptacle, it is to be understood that module 260 may be used with any receptacle having an aperture sufficient in size to penetrably receive shroud 242 and permit locking the module in place with a plate, such as plate 260.

Blood collection holder device 50, illustrated in Figures 1, 3 and 5, was developed by Medical Safety Products, Inc. for the purpose of eliminating exposure of both the anterior and posterior needles of a double ended needle while handling of the holder other than during the venipuncture procedure itself. That is, after attachment of a conventional double ended needle, the device may be immediately used for venipuncture; alternatively, the double ended needle may be retracted within the guard of the device in the event there will be a time lapse prior to use. After withdrawal of the needle on completion of a venipuncture procedure, the anterior needle is retracted within the guard to prevent needle stick and to contain any body fluids of the patient which may be on or about the needle. Prior to retraction, the posterior needle is shielded by both the holder and the guard. Upon retraction of the double ended needle, the posterior needle remains shielded within the holder to prevent needle stick or contact therewith by a clinician. Passageway 90 (Figure 3) or the slot adjacent cylindrical wall 170 (Figure 10) receives the collar extending anteriorly from the barrel, which collar may partly house and shield the end of the anterior needle. Prior to and during the act of mating the collar with the passageway or the slot, the anterior needle remains shielded and accidental contact therewith by a clinician will be precluded. During extension of the double ended needle prior to segregation of the needle from the holder, the anterior needle is interior of receptacle 10 (Figure 1), which receptacle shields the needle against accidental contact by the clinician. After unthreading of the double ended needle from the holder, the needle will drop into the receptacle with little possibility that the clinician can contact either the anterior or posterior needle of the double ended needle. Accordingly, the combination of device 50 and receptacle 10 provides apparatus which will completely safeguard a clinician from contact with a double ended needle during the time subsequent to a venipuncture procedure to final disposal of the double ended needle.

While the principles of the invention have now been made clear in an illustrative embodiment, there will be immediately obvious to those skilled in the art many modifications of structure, arrangement, proportions, elements, materials and components used in the practice of the invention which are particularly adapted for specific environments and operating requirements without departing from those principles.

## Claims

1. A disposal for receiving double ended needles (48, 62) of blood collection holders (34,50,54), in a receptacle (10), said disposal comprising in combination:
- at least one blood collection holder (34,50,54) provided with a said double ended needle (48,62) having a hub (44, 60) threadedly engaged with a boss (46, 56) of said blood collection holder (34,50, 54);
- a receptacle (10) housing separated needles (48,62);
- a receiving means (30,90,234) disposed in said receptacle (10) receiving the anterior end (32, 58) of a blood collection tube holder (34,50, 54) and accommodating rotation of the holder (34,50,54) about its longitudinal axis, which holder (34,50,54) includes a boss (46,56) engaging said hub (44,60)
- a stationary engaging means (38,40,92,94,138, 198,236,238) disposed within said receiving means (30,90,234) engaging the hub (44,60) to prevent rotation of the hub (44,60) about the longitudinal axis of the needle (48,62) during rotation of said holder (34,50,54) and thereby separate the needle (48, 62) from the holder (34,50,54); and
- an aperture (39,90) disposed in said receiving means (30,90,234) accommodating passage of the needle (48,62) upon disengagement of the needle (48,62) from the holder (34,50,54) resulting from rotation of the holder (34,50,54) relative to the hub (44,60) and withdrawal of the holder (34,50,54) from said receiving means (30,90),
**characterized by**
- a bias means which comprises at least one leaf spring (110,142,144,178,180) and actively urges the separated needle (48,62) into said receptacle (10).

2. Disposal as set forth in claim 1, wherein said hub (44,60) has a rib (42,61) and wherein said engaging means comprises a slot (40,92,238) disposed in a post (38,94,236), the slot (40,92,238) engaging a rib (42,61) of the ribbed hub (44, 60).

3. Disposal as set forth in claim 2, wherein said leaf spring (110) extends from said post (38,94).

4. Disposal as set forth in claim 1, wherein said hub (44,60) has a rib (42,61) and wherein said engaging means comprises a post (138,198), said post (138, 198) interferingly engaging a rib (42,61) of the ribbed hub (44,60).

5. Disposal as set forth in one of the claims 1 to 4, wherein said receiving means is comprised of a recess (30) including a means (36) supporting at least a part of the anterior end (32) of said holder (34), said supporting means including a segment of an annular surface.

6. Disposal as set forth in one of the claims 1 to 5, wherein said leaf spring (110,142,144,178,180) includes a lip (122, 152,154,186,188) for engaging the hub (44,60) to prevent rise of the disengaged needle (48,62) upon withdrawal of the holder (34,54) from said recess (30,90).

7. Disposal as set forth in one of the claims 1 to 6, wherein said blood collection holder (50) includes a barrel (52) receiving and supporting a holder (54) and having an anterior collar (78), said holder (54) being translatable and having a boss (56) for threadedly engaging said needle hub (60);
and wherein said receptacle (10) has a passageway (90) to receive and guide said collar (78) of said barrel (52) and to accept the anterior end (74) of said needle (62) into the interior of said receptacle (10) as said holder (54) is moved anteriorly.

8. Disposal as set forth in one of the claims 1 to 7, wherein said holder (34,50,54) is transparent to permit viewing disengagement of the needle (48,62) from the holder (34,50,54).

9. Disposal as set forth in one of the claims 1 to 8, wherein said receptacle (10) includes means to permit viewing drop of the needle (48,62) into said receptacle (10).

10. Disposal as set forth in one of the claims 1 to 9, wherein an aperture (252) receives a module (230) having said receiving means (234) said engaging means (236, 238) and said bias means (110,142,144, 178,180) formed therein.

11. A method for disposing double ended needles (48,62) of a blood collection holder (34,54), said method comprising the steps of:
a) providing a receptacle (10) for housing the needles (48,62);
b) receiving the anterior end (32,58) of said blood collection tube holder (34,54) in a recess (30, 90,234) associated with the receptacle (10), which recess (30,90,234) accommodates rotation of the holder (34,54) about its longitudinal axis and supports at least a part of the anterior end (32,58) of the holder (34,54), the holder (34,54) including a boss (46,56) at the anterior end (32,58) for threadedly engaging a hub (44, 60) disposed upon the needle (48,62);
c) interferingly engaging said hub (44,60) upon attempted rotation of the hub (44,60) with a stationary engaging means (38,40,92,94,138,198, 236,238) disposed in the recess (30,90,234) to prevent rotation of the hub (44,60) about the longitudinal axis of the needle (48,62) during rotation of said holder (34,54), thereby unthreading the needle (48,62) from said holder (34,54), and
**characterized by**
d) passing the needle (48,62) through an aperture (39,90) disposed in the recess (30,90) and into the receptacle (10) upon threaded disengagement of the needle (48,62) from the holder (34,54) by actively urging the unthreaded needle (48,62) into said receptacle (10) by a bias means comprising at least one leaf spring (110,142,144, 178,180).

12. The method as set forth in claim 11 including the step of restraining rise of the needle (48,62) upon withdrawal of the holder (34,54).

## Patentansprüche

1. Entsorgungsvorrichtung, um doppelendige Nadeln (48,62) von Blutsammelhaltern (34,50,54) in ein Aufnahmebehältnis (10) einzuführen, wobei die Entsorgungsvorrichtung in Kombination aufweist:
- mindestens einen Blutsammelhalter (34,50,54) mit einer doppelendigen Nadel (48,62), die einen in Gewindeeingriff mit einem Vorsprung (46,56) des Blutsammelhalters (34,50,54) befindlichen Ansatz (44,60) aufweist,
- ein Aufnahmebehältnis (10) zur Unterbringung abgetrennter Nadeln (48,62),
- eine in dem Aufnahmebehältnis (10) angeordnete Aufnahmeeinrichtung (30,90,234), die das Vorderende (32,58) eines Blutsammelröhrchenhalters (34,50,54) aufnimmt und eine Drehung des Halters (34,50,54) um seine Längsachse erlaubt, wobei der Halter (34,50,54) einen mit dem Ansatz (44,60) zusammengreifenden Vorsprung (46,56) aufweist,
- eine innerhalb der Aufnahmeeinrichtung (30,90,234) angeordnete stationäre Angreifeinrichtung (38,40,92,94, 138,198,236,238), die derart mit dem Ansatz (44,60) zusammengreift, daß während einer Drehung des Halters (34,50,54) eine Drehung des Ansatzes (44,60) um die Längsachse der Nadel (48,62) verhindert und dadurch die Nadel (48,62) von dem Halter (34,50,54) getrennt wird, und
- eine in der Aufnahmeeinrichtung (30,90,234) ausgebildete Öffnung (39,90), die bei dem Ausrücken der Nadel (48,62) aus dem Halter (34,50,54), das durch die Drehung des Halters (34,50,54) relativ zu dem Ansatz (44,60) und das Abziehen des Halters (34,50,54) von der Aufnahmeeinrichtung (30,90) verursacht wird, die Nadel (48,62) hindurchläßt,
**gekennzeichnet durch**
- eine Vorspanneinrichtung, die mindestens eine Blattfeder (110,142,144,178,180) aufweist und die abgetrennte Nadel (48,62) aktiv in das Aufnahmebehältnis (10) drückt.

2. Entsorgungsvorrichtung nach Anspruch 1, bei der der Ansatz (44,60) eine Rippe (42,61) aufweist und bei der die Angreifeinrichtung einen in einem Stab (38,94,236) ausgebildeten Spalt (40,92,238) aufweist, der mit einer Rippe (42,61) des gerippten Ansatzes (44,60) zusammengreift.

3. Entsorgungsvorrichtung nach Anspruch 2, bei der die Blattfeder (110) von dem Stab (38,94) absteht.

4. Entsorgungsvorrichtung nach Anspruch 1, bei der der Ansatz (44,60) eine Rippe (42,61) aufweist und bei der die Angreifeinrichtung einen Stab (138,198) aufweist, der blokkierend an eine Rippe (42,61) des gerippten Ansatzes (44, 60) angreift.

5. Entsorgungsvorrichtung nach einem der Ansprüche 1 bis 4, bei der die Aufnahmeeinrichtung eine Vertiefung (30) mit einer Einrichtung (36) aufweist, welche mindestens einen Teil des Vorderendes (32) des Halters (34) stützt, wobei die Stützeinrichtung ein Segment einer Ringfläche aufweist.

6. Entsorgungsvorrichtung nach einem der Ansprüche 1 bis 5, bei der die Blattfeder (110,142,144,178,180) eine Lippe (122,152,154,186,188) aufweist, die an den Ansatz (44,60) angreift, um beim Abziehen des Halters (34,54) von der Vertiefung (30,90) eine Aufwärtsbewegung der abgetrennten Nadel (48,62) zu verhindern.

7. Entsorgungsvorrichtung nach einem der Ansprüche 1 bis 6, bei der der Blutsammelhalter (50) ein Gefäß (52) aufweist, das einen Behälter (54) aufnimmt und stützt und mit einem vorderen Kragen (78) versehen ist, wobei der Behälter (54) translatorisch bewegbar ist und einen Vorsprung (56) zum Gewindeeingriff mit dem Nadelansatz (60) aufweist,
und wobei das Aufnahmebehältnis (10) einen Durchlaß (90) aufweist, der den Kragen (78) des Gefäßes (52) aufnimmt und führt und das Vorderende (74) der Nadel (62) in das Innere des Aufnahmebehältnisses (10) einläßt, wenn der Behälter (54) vorbewegt wird.

8. Entsorgungsvorrichtung nach einem der Ansprüche 1 bis 7, bei der der Halter (34,50,54) durchsichtig ist, um eine Sichtkontrolle des Ausrückens der Nadel (48,62) aus dem Halter (34,50,54) zu ermöglichen.

9. Entsorgungsvorrichtung nach einem der Ansprüche 1 bis 8, bei der das Aufnahmebehältnis (10) mit einer Einrichtung versehen ist, die eine Sichtkontrolle des Hinabfallens der Nadel (48,62) in das Aufnahmebehältnis (10) ermöglicht.

10. Entsorgungsvorrichtung nach einem der Ansprüche 1 bis 9, bei der eine Öffnung (252) ein Modul (230) aufnimmt, in dem die Aufnahmeeinrichtung (234), die Angreifeinrichtung (236,238) und die Vorspanneinrichtung (110,142,144,178, 180) ausgebildet sind.

11. Verfahren zum Entsorgen doppelendiger Nadeln (48,62) eines Blutsammelhalters (34,54), mit den folgenden Schritten:
a) Bereitstellen eines Aufnahmebehältnisses (10) zum Unterbringen der Nadeln (48,62),
b) Aufnehmen des Vorderendes (32,58) des Blutsammelröhrchenhalters (34,54) in einer dem Aufnahmebehältnis (10) zugeordneten Vertiefung (30,90,234), die eine Drehung des Halters (34,54) um seine Längsachse erlaubt und mindestens einen Teil des Vorderendes (32, 58) des Halters (34,54) stützt, wobei der Halter (34, 54) an dem Vorderende (32,58) einen Vorsprung (46,56) zum Gewindeeingriff mit einem an der Nadel (48,62) angeordneten Ansatz (44,60) aufweist,
c) mittels einer in der Vertiefung (30,90,234) angeordneten stationären Angreifeinrichtung (38,40,92,94, 138,198,236,238) erfolgendes blockierendes Angreifen an den Ansatz (44,60) bei versuchter Drehung des Ansatzes (44,60), um während einer Drehung des Halters (34,54) eine Drehung des Ansatzes (44,60) um die Längsachse der Nadel (48,62) zu verhindern und dadurch die Nadel (48,62) von dem Halter (34,54) abzuschrauben, und
**gekennzeichnet durch**
d) Durchführen der Nadel (48,62) durch eine in der Vertiefung (30,90) ausgebildete Öffnung (39,90) und in das Aufnahmebehältnis (10) bei Lösen des Gewindeeingriffs zwischen der Nadel (48,62) und dem Halter (34, 54), indem die abgeschraubte Nadel (48,62) mittels einer mindestens eine Blattfeder (110,142,144,178,180) aufweisenden Vorspanneinrichtung aktiv in das Aufnahmebehältnis (10) gedrückt wird.

12. Verfahren nach Anspruch 11, mit dem Schritt des Verhinderns einer Aufwärtsbewegung der Nadel (48,62) beim Abziehen des Halters (34,54).

## Revendications

1. Poubelle de réception d'aiguilles à double extrémité (48, 62), de récipients (34, 50, 54) de collecte de sang, dans une cuve (10), ladite poubelle comprenant en combinaison :
- au moins un récipient (34, 50, 54) de collecte de sang muni d'une dite aiguille (48, 62) à double extrémité comportant un moyeu (44, 60) en prise par filetage avec une protubérance (46, 56) dudit récipient (34, 50, 54) de collecte de sang ;
- une cuve (10) servant de logement aux aiguilles détachées (48, 62) ;
- un moyen de réception (30, 90, 234) ménagé dans ladite cuve (10) pour recevoir l'extrémité antérieure (32, 58) d'un récipient tubulaire (34, 50, 54) de collecte de sang et pour permettre la rotation du récipient (34, 50, 54) autour de son axe longitudinal, lequel récipient (34, 50, 54) comprend une protubérance (46, 56) en prise avec ledit moyeu (44, 60),
- un moyen fixe de venue en prise (38, 40, 92, 94, 138, 198, 236, 238) disposé dans ledit moyen de réception (30, 90, 234) pour venir en prise avec le moyeu (44, 60) afin d'empêcher la rotation du moyeu (44, 60) autour de l'axe longitudinal de l'aiguille (48, 62) pendant la rotation dudit récipient (34, 50, 54) et pour séparer ainsi l'aiguille (48, 62) du récipient (34, 50, 54) ; et
- un orifice (39, 90) ménagé dans ledit moyen de réception (30, 90, 234) permettant le passage de l'aiguille (48, 62) lors de la séparation de l'aiguille (48, 62) du récipient (34, 50, 54) résultant de la rotation du récipient (34, 50, 54) par rapport au moyeu (44, 60) et du retrait du récipient (34, 50, 54) à partir du moyen de réception (30, 90),
caractérisé par un moyen de sollicitation qui comprend au moins un ressort à lame (110, 142, 144, 178, 180) et qui pousse activement l'aiguille détachée (48, 62) dans ladite cuve (10).

2. Poubelle telle que définie dans la revendication 1, dans laquelle ledit moyeu (44, 60) comporte une nervure (42, 61) et dans laquelle ledit moyen de venue en prise comprend une fente (40, 92, 238) ménagée dans un montant (38, 94, 236), la fente (40, 92, 238) venant en prise avec une nervure (42, 61) du moyeu (44, 60) nervuré.

3. Poubelle telle que définie dans la revendication 2 dans laquelle le ressort à lame (110) s'étend à partir dudit montant (38, 94).

4. Poubelle telle que définie dans la revendication 1, dans laquelle ledit moyeu (44, 60) comporte une nervure (42, 61) et dans laquelle ledit moyen de venue en prise comprend un montant (138, 198) ledit montant (138, 198) venant en prise d'interférence avec une nervure (42, 61) du manchon nervuré (44, 60).

5. Poubelle telle que définie dans l'une des revendications 1 à 4, dans laquelle ledit moyen de réception est composé d'un renfoncement (30) comprenant un moyen (36) pour supporter au moins une partie de l'extrémité antérieure (32) dudit récipient (34), ledit moyen de support comprenant un segment de surface annulaire.

6. Poubelle telle que définie dans l'une des revendications 1 à 5, dans laquelle ledit ressort à lame (110, 142, 144, 178, 180) comprend une lèvre (122, 152, 154, 186, 188) pour venir en prise avec le moyeu (44, 60) afin d'empêcher le soulèvement de l'aiguille détachée (48, 62) lors du retrait du récipient (34, 54) à partir du renfoncement (30, 90).

7. Poubelle telle que définie dans l'une des revendications 1 à 6, dans laquelle ledit récipient (50) de collecte de sang comprend un fût (52) de réception et de support d'un récipient (54) et comportant une collerette antérieure (78), ledit récipient (54) étant mobile en translation et comportant une protubérance (56) pour venir en prise par un filetage avec ledit moyeu (60) de l'aiguille ; et dans laquelle ladite cuve (10) comporte un passage (90) pour recevoir et guider ladite collerette (78) dudit fût (52) et pour recevoir l'extrémité antérieure (74) de ladite aiguille (62) à l'intérieur de ladite cuve (10) tandis que ledit récipient (54) est déplacé vers l'avant.

8. Poubelle telle que définie dans l'une des revendications 1 à 7, dans laquelle ledit récipient (34, 50, 54) est transparent pour permettre d' observer la séparation de l'aiguille (48, 62) vis'à-vis du récipient (34, 50, 54).

9. Poubelle telle que définie dans l'une des revendications 1 à 8, dans laquelle ladite cuve (10) comprend un moyen pour permettre de voir la chute de l'aiguille (48, 62) dans ladite cuve (10).

10. Poubelle telle que définie dans l'une des revendications 1 à 9 dans laquelle un orifice (252) reçoit un module (230) comportant ledit moyen de réception (234), ledit moyen de venue en prise (236, 238) et ledit moyen de sollicitation (110, 142, 144, 178, 180) formés dans ce module.

11. Méthode pour se débarrasser d'aiguilles (48, 62) à double extrémité d'un récipient (34, 54) de collecte de sang, ladite méthode comprenant les étapes consistaat à :
a) prévoir une cuve (10) pour loger les aiguilles (48, 62);
b) recevoir l'extrémité antérieure (32, 58) dudit récipient tubulaire (34, 54) de collecte de sang dans un renfoncement (30, 90, 234) associé à la cuve (10), lequel renfoncement (30, 90, 234) permet la rotation du récipient (34, 54) autour de son axe longitudinal et supporte au moins une partie de l'extrémité antérieure (32, 58) du récipient (34, 54), le récipient (34, 54) comprenant une protubérance (46, 56) à l'extrémité antérieure (32, 58) pour venir en prise par filetage avec un moyeu (44, 60) ménagé sur l'aiguille (48, 62);
c) mettre en prise d'interférence ledit moyeu (44, 60) lors d'une tendance à la rotation du moyeu (44, 60) avec un moyen fixe de venue en prise (38, 40, 92, 94, 138, 198, 236, 238) ménagé dans le renfoncement (30, 90, 234) pour empêcher la rotation du moyeu (44, 60) autour de l'axe longitudinal de l'aiguille (48, 62) pendant la rotation dudit récipient (34, 54), et pour dévisser ainsi l'aiguille (48, 62) dudit récipient (34, 54), et
caractérisée par l'étape consistant à:
d) faire passer l'aiguille (48, 62) à travers un orifice (39, 90), ménagé dans le renfoncement (30, 90), et dans la cuve (10) lors de la séparation du filetage de l'aiguille (48, 62) vis-à-vis du récipient (34, 54) en poussant activement l'aiguille (48, 62) dévissée dans ladite cuve (10) par un moyen de sollicitation comprenant au moins au ressort à lame (110, 142, 144, 178, 180).

12. Méthode telle que définie dans la revendication 11 comprenant l'étape consistant à empêcher le soulèvement de l'aiguille (48, 62) lors du retrait du récipient (34, 54).
